# EUROPEAN PATENT APPLICATION

(11) **EP 4 691 472 A1**
(43) Date of publication of application: **11.02.2026**
(21) Application number: 24784993.8
(22) Date of filing: 05.04.2024
(51) Int. Cl.: A61K 31/662, A61P 25/28

(54) **IMPROVING/THERAPEUTIC DRUG FOR DEMENTIA, PARTICULARLY ALZHEIMER-TYPE DEMENTIA**

(30) Priority: 06.04.2023 JP 2023061827
(71) Applicant: Homo Contribuens Research and Development Institute, Tokyo 100-0006 (JP); Tsubasa Pharma Co., Ltd., Saitama-shi, Saitama 336-0026 (JP); Murofushi, Kimiko, Tokyo 170-0013 (JP)
(72) Inventor: MUROFUSHI, Wataru, Tokyo 170-0003 (JP); MUROFUSHI, Kimiko, Tokyo 170-0013 (JP)
(74) Representative: Godemeyer Blum Lenze Patentanwälte Partnerschaft mbB - werkpatent
(86) International application number: PCT/JP2024/014133
(87) International publication number: WO 2024/210211

(57) **Abstract**

For dementia, particularly Alzheimer's disease, there is currently no effective agent for ameliorating or treating it. The object of the present invention is to find a drug for ameliorating or treating dementia thereby providing a novel agent for ameliorating or treating dementia. The present invention provides an agent for ameliorating or treating dementia, which comprises cyclic phosphatidic acid, carbacyclic phosphatidic acid, thiacyclic phosphatidic acid, or carba-lysophosphatidic acid, or a salt thereof.

## Description

### Technical Field

The present invention relates to an agent for ameliorating or treating dementia, primarily Alzheimer's dementia, which comprises cyclic phosphatidic acid, carbacyclic phosphatidic acid, thiacyclic phosphatidic acid, or carba-lysophosphatidic acid as an active ingredient.

### Background Art

In 1992, Kimiko Murofushi et al., the present inventors, found, isolated, and purified a lipid-soluble substance from the haploid myxoamoeba of the true slime mold *Physarum polycephalum* that inhibits the activity of DNA polymerase α, which is a DNA replication enzyme in eukaryotic cells, and suppresses the growth of cultured animal cells (Non-Patent Document 1). This substance was found to have a hexadecanoic acid containing cyclopropane bonded to the *sn*-1 position of the glycerol skeleton, and phosphoric acid bonded to the 2nd and 3rd positions via cyclic ester bonds. As it is an LPA-like substance derived from *Physarum,* it was named PHYLPA. Since PHYLPA has a characteristic fatty acid at the *sn*-1 position, the inventors chemically synthesized a derivative thereof with a common fatty acid substitution and examined the activity thereof. As a result, it was shown that the derivative inhibits cell proliferation, and it became clear that the growth-inhibiting effect of PHYLPA is due to the cyclic phosphate groups at the 2nd and 3rd positions. Currently, such LPA analogs having cyclic phosphate groups are collectively referred to as cyclic phosphatidic acids (cPAs).

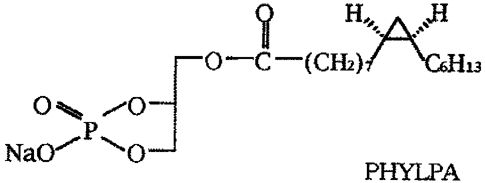

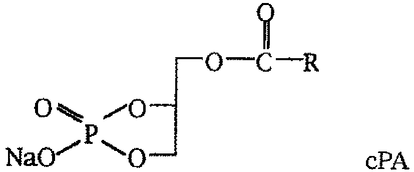

Regarding cyclic phosphatidic acids and their derivatives, there have been reports on their neurotrophic factor action and application to neurodegenerative diseases (Patent Documents 1 and 2), inhibition of cancer cell proliferation, invasion, and metastasis (Patent Document 3), analgesic action (Patent Document 4), application to atopic dermatitis (Patent Document 5), application to demyelinating diseases (Patent Document 6 and Non-Patent Document 2), and injury treatment (Patent Document 7). However, there is no finding about their effects for ameliorating or treating dementia, primarily Alzheimer's dementia. As shown in Patent Document 8, a new cyclic phosphatidic acid analog, 2cLPA, was identified and reported to have activity similar to that of cyclic phosphatidic acid; however, there is no finding available about its effect of in ameliorating or treating dementia, primarily Alzheimer's dementia.

### Prior Art Documents

### Patent Documents

Patent Document 1: JP 2002-308778 A
Patent Document 2: JP 2002-308779 A
Patent Document 3: WO 2002/94286
Patent Document 4: WO 2008/81580
Patent Document 5: JP 2012-56853 A
Patent Document 6: WO 2014/115885
Patent Document 7: JP 2018-080118 A
Patent Document 8: JP 2021-80227 A

### Non-Patent Documents

Non-Patent Document 1: Murakami-Murofushi,K., et al., J. Biol. Chem. 267,21512-21517 (1992)
Non-Patent Document 2: Yamamoto, S., et al., European Journal of Pharmacology 741 (2014) 17-24

### Summary of Invention

### Object to be Solved by the Invention

In Japan, which is now a super-aged society and is said to be a country facing advanced challenges, there is a large gap between average life expectancy and healthy life expectancy, and dementia, especially among the elderly, has become a major social issue. To date, there are no effective agents for ameliorating or treating dementia, primarily Alzheimer's dementia. If drugs that can ameliorate or treat these dementias can be found, it will be possible to develop novel agents for ameliorating or treating these conditions. Moreover, the present inventors have been conducting research into cyclic phosphatidic acid, carbacyclic phosphatidic acid, thiacyclic phosphatidic acid, and carba-lysophosphatidic acid, which are low-molecular-weight substances that are easy to handle, highly safe, and can be produced at low cost. Therefore, these substances are expected to have great potential as drugs that can slow the progression of dementia, primarily Alzheimer's dementia, and ameliorate or treat the symptoms. The goal of this development is to extend people's healthy lifespans and improve their quality of life, while at the same time preventing the collapse of the country's medical economy.

### Means for Solving the Object

The present inventors found that cyclic phosphatidic acid, carbacyclic phosphatidic acid, thiacyclic phosphatidic acid, or carba-lysophosphatidic acid and their derivatives have the effect of ameliorating and treating dementia, primarily Alzheimer's dementia. This has led to the completion of the present invention.

The present invention provides an agent for ameliorating or treating dementia, which comprises cyclic phosphatidic acid, carbacyclic phosphatidic acid, thiacyclic phosphatidic acid, or carba-lysophosphatidic acid, or a salt thereof.

Preferably, the dementia is Alzheimer's dementia.

Preferably, the cyclic phosphatidic acid, carbacyclic phosphatidic acid, or thiacyclic phosphatidic acid, or a salt thereof is a compound represented by Formula (1). Preferably, the carba-lysophosphatidic acid or a salt thereof is a compound represented by Formula (2). 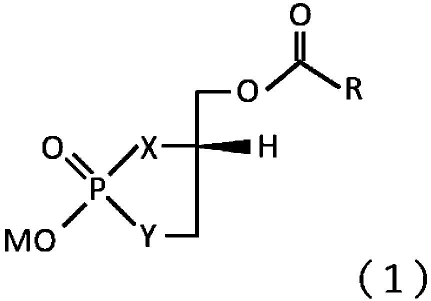 (in Formula (1), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring; X and Y each independently represent -O-, -S- or -CH₂-, provided that X and Y are not simultaneously -CH₂-; and M is a hydrogen atom or a counter cation) 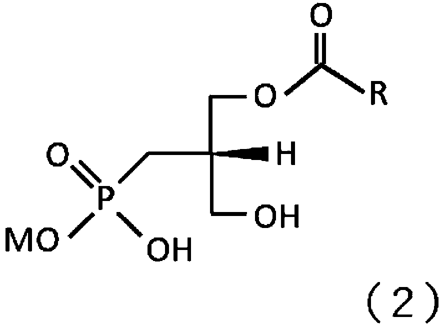 (in Formula (2), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring; and M is a hydrogen atom or a counter cation).

Preferably, one of X and Y is -CH₂-, and the other of X and Y is -O- in Formula (1).

The present invention provides a method for ameliorating or treating dementia, which comprises administering cyclic phosphatidic acid, carbacyclic phosphatidic acid, thiacyclic phosphatidic acid, or carba-lysophosphatidic acid, or a salt thereof to a patient suffering from dementia.

The present invention further provides use of cyclic phosphatidic acid, carbacyclic phosphatidic acid, thiacyclic phosphatidic acid, or carba-lysophosphatidic acid, or a salt thereof, for production of an agent for treating dementia.

The present invention further provides cyclic phosphatidic acid, carbacyclic phosphatidic acid, thiacyclic phosphatidic acid, or carba-lysophosphatidic acid, or a salt thereof for use in amelioration or treatment of dementia.

### Advantageous Effects of Invention

According to the present invention, a therapeutic agent for dementia can be provided, which contains cyclic phosphatidic acid, carbacyclic phosphatidic acid, thiacyclic phosphatidic acid, or carba-lysophosphatidic acid as an active ingredient.

### Brief Description of Drawings

Fig. 1 shows the results of the Morris water maze test performed on normal mice, pathological mice, and pathological mice administered with the test substances (2ccPA, 2cLPA) for 24 weeks.
Fig. 2 is a line graph showing the results of administering low doses of 2ccPA and 2cLPA, which demonstrated clear effects in the Morris water maze test.

### Embodiment of Carrying out the Invention

Hereinafter, the present invention will be described in more detail.

The present invention relates to an agent for treating dementia, primarily Alzheimer's dementia, which contains cyclic phosphatidic acid, carbacyclic phosphatidic acid, thiacyclic phosphatidic acid, carba-lysophosphatidic acid, or a salt thereof as an active ingredient. The agent for treating dementia of the present invention can be used to ameliorate or treat dementia, and contains cyclic phosphatidic acid, carbacyclic phosphatidic acid, thiacyclic phosphatidic acid, carba-lysophosphatidic acid, or a salt thereof as an active ingredient. There are no particular limitations on the cyclic phosphatidic acid, carbacyclic phosphatidic acid, thiacyclic phosphatidic acid, or carba-lysophosphatidic acid, as long as it exhibits the effects of the present invention. Preferably, cyclic phosphatidic acid, represented by the following Formula (1), or carba-lysophosphatidic acid, represented by the following Formula (2), can be used.

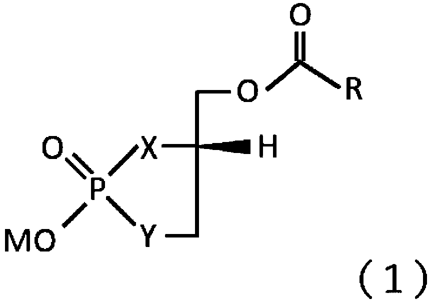

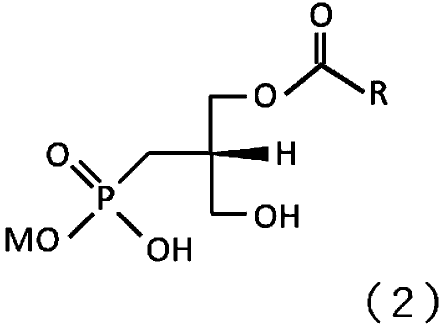

In Formula (1), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring. X and Y each independently represent -O-, -S- or -CH₂-, provided that X and Y are not simultaneously -CH₂-. M is a hydrogen atom or a counter cation.

In Formula (2), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring. M is a hydrogen atom or a counter cation.

In Formulas (1) and (2), specific examples of the linear or branched alkyl group having 1 to 30 carbon atoms represented by the substituent R include, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an eicosyl group.

Specific examples of the linear or branched alkenyl group having 2 to 30 carbon atoms represented by the substituent R include an allyl group, a butenyl group, an octenyl group, a decenyl group, a dodecadienyl group, and a hexadecatrienyl group. More specific examples include an 8-decenyl group, an 8-undecenyl group, an 8-dodecenyl group, an 8-tridecenyl group, an 8-tetradecenyl group, an 8-pentadecenyl group, an 8-hexadecenyl group, an 8-heptadecenyl group, an 8-octadecenyl group, an 8-icosenyl group, an 8-docosenyl group, a heptadeca-8,11-dienyl group, a heptadeca-8,11,14-trienyl group, a nonadeca-4,7,10,13-tetraenyl group, a nonadeca-4,7,10,13,16-pentaenyl group, and a henicosa-3,6,9,12,15,18-hexaenyl group.

Specific examples of the linear or branched alkynyl group having 2 to 30 carbon atoms represented by the substituent R include an 8-decynyl group, an 8-undecynyl group, an 8-dodecynyl group, an 8-tridecynyl group, an 8-tetradecynyl group, an 8-pentadecynyl group, an 8-hexadecynyl group, an 8-heptadecynyl group, an 8-octadecynyl group, an 8-icosynyl group, an 8-docosynyl group, and a heptadeca-8,11-diynyl group.

Specific examples of the cycloalkane ring that can be included in the alkyl group, alkenyl group, or alkynyl group include a cyclopropane ring, a cyclobutane ring, a cyclopentane ring, a cyclohexane ring, and a cyclooctane ring. The cycloalkane ring may include one or more heteroatoms, and examples thereof include an oxirane ring, an oxetane ring, a tetrahydrofuran ring, and an N-methylprolidone ring.

Specific examples of the aromatic ring that can be included in the alkyl group, alkenyl group, or alkynyl group include a benzene ring, a naphthalene ring, a pyridine ring, a furan ring, and a thiophene ring.

Therefore, specific examples of the substituent R which is an alkyl group substituted with a cycloalkane ring include a cyclopropylmethyl group, a cyclohexylethyl group, and an 8,9-methanopentadecyl group.

Specific examples of the substituent R which is an alkyl group substituted with an aromatic ring include a benzyl group, a phenethyl group, and a p-pentylphenyloctyl group.

R is preferably a linear or branched alkyl group having 9 to 17 carbon atoms, a linear or branched alkenyl group having 9 to 17 carbon atoms, or a linear or branched alkynyl group having 9 to 17 carbon atoms. R is more preferably a linear or branched alkyl group having 9, 11, 13, 15, or 17 carbon atoms, or a linear or branched alkenyl group having 9, 11, 13, 15, or 17 carbon atoms. R is particularly preferably a linear or branched alkenyl group having 9, 11, 13, 15, or 17 carbon atoms.

In the compound represented by Formula (1), X and Y each independently represent -O-, -S- or -CH₂-, provided that X and Y are not simultaneously -CH₂-. That is, there are three combinations of X and Y as follows:
(1) X is -O- and Y is -O-;
(2) X is -CH₂- and Y is -O- (2-carba-cPA (also abbreviated as 2ccPA)) or X is -S- and Y is -O-; and
(3) X is -O- and Y is -CH₂- (3-carba-cPA (also abbreviated as 3ccPA)) or X is A and Y is S.

Of the above, a compound in which X is -CH₂- and Y is -O- (2-carba-cPA) is particularly preferred.

M in the cyclic phosphatidic acid derivatives represented by Formulas (1) and (2) is a hydrogen atom or a counter cation. When M is a counter cation, examples thereof include an alkali metal atom, an alkaline earth metal atom, and a substituted or unsubstituted ammonium group. Examples of an alkali metal atom include lithium, sodium, and potassium, and examples of an alkaline earth metal atom include magnesium and calcium. Examples of a substituted ammonium group include a butylammonium group, a triethylammonium group, and a tetramethylammonium group.

The compounds of Formulas (1) and (2) may have isomers such as positional isomers, geometric isomers, tautomers, or optical isomers, depending on the types of substituents. All possible isomers and mixtures containing two or more of the isomers in any ratio are within the scope of the present invention.

Furthermore, the compounds of Formulas (1) and (2) may exist in the form of adducts (hydrates or solvates) with water or various solvents, and these adducts are also within the scope of the present invention. Moreover, any crystalline forms of the compound of Formula (1) and a salt thereof are also within the scope of the present invention.

Preferably, the compound represented by Formula (1) is 1-oleoyl cyclic phosphatidic acid or 1-palmitoleoyl cyclic phosphatidic acid. Specific examples of the compound represented by Formula (1) used in the present invention include oleoyl-2-carba-cPA (ΔOle-2ccPA) and palmitoleoyl-2-carba-cPA (ΔPal-2ccPA). In addition, preferably, the compound represented by Formula (2) is 1-oleoylcarbalizophosphatidic acid or 1-palmitoleoylcarbalizophosphatidic acid. Specific examples of the compound represented by Formula (2) used in the present invention include oleoyl-2-carba-LPA (ΔOle-2cLPA) and palmitoleoyl-2-carba-LPA (ΔPal-2cLPA).

Among the compounds represented by Formula (1), a compound in which X and Y are -O- can be synthesized in accordance with the methods described, for example, in Kobayashi, S., et al.: Tetrahedron Lett., 34, 4047-4050 (1993), JP 5-230088 A (1993), JP 7-149772 A (1995), JP 7-258278 A (1995), and JP 9-25235 A (1997). In addition, the compound represented by Formula (2) can be synthesized according to the method described in JP 2021-80227 A.

Furthermore, among the compounds represented by Formula (1), a compound in which X and Y are -O- can also be synthesized by reacting lysophospholipids with phospholipase D in accordance with the method described in JP 2001-178489 A. The lysophospholipids used herein are not particularly limited as long as they are lysophospholipids on which phospholipase D can act. Many types of lysophospholipids are known, including those with different fatty acid species and molecular species with ether or vinyl ether bonds, and these are available as commercial products. Phospholipase D derived from higher plants such as cabbage and peanut, and from microorganisms such as *Streptomyces chromofuscus* and *Actinomadula sp.,* is available as a commercial reagent; however, cPA is synthesized very selectively by the enzyme derived from *Actinomadula sp.* No.362 (JP 11-367032 A (1999)). The reaction between a lysophospholipid and phospholipase D is not particularly limited as long as the conditions allow the enzyme to express its activity. For example, the reaction can be carried out in an acetate buffer solution (pH about 5 to 6) containing calcium chloride at room temperature or under elevated temperatures (preferably about 37°C) for about 1 to 5 hours. The resulting cPA derivative can be purified in accordance with an ordinary methods, such as extraction, column chromatography, or thin-layer chromatography (TLC).

Furthermore, among the compounds represented by Formula (1), a compound in which X or Y is -S- can be synthesized according to the description in Bioorganic & Medicinal Chemistry Letters 21 (2011) 4180-4182 or Bioorganic & Medicinal Chemistry 20 (2012) 3196-3201.

Furthermore, among the compounds represented by Formula (1), a compound in which X is -CH₂- and Y is -O- can be synthesized by the method described in JP 2004-010582 A or WO 03/104246.

Furthermore, among the compounds represented by Formula (1), a compound in which X is -O- and Y is -CH₂- can be synthesized in accordance with the methods described in the literature (Uchiyama A. et al., Biochimica et Biophysica Acta 1771 (2007) 103-112; and "The 23rd Symposium on Progress in Organic Reactions and Syntheses Symposium of the Pharmaceutical Society of Japan, November 17 and 18, 1997 (Kumamoto Civic Hall), Synthesis and Physiological Activities of Cyclic Phosphatidic Acid and Carba Derivatives, Abstracts, pp. 101-104"), or can be synthesized by the method described in WO 2002/094286. An example of a specific synthetic route is shown below.

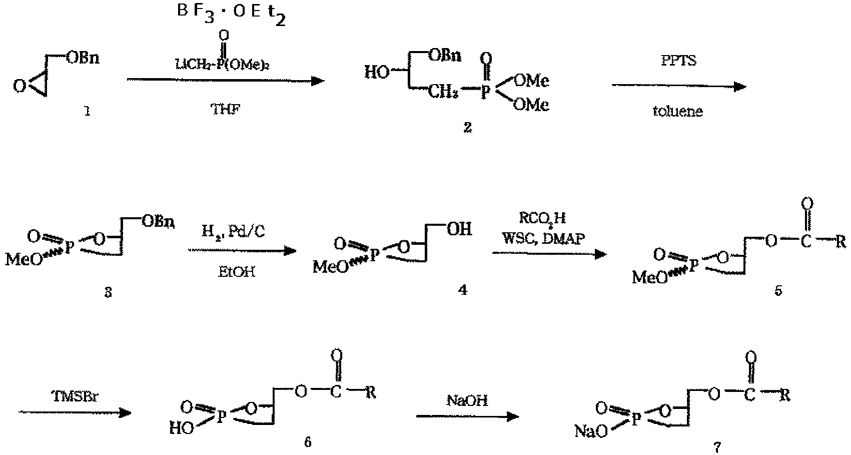

In the above process, commercially available (R)-benzyl glycidyl ether (1) is first activated with BF₃ • Et₂O, and the lithio form obtained by reacting methylphosphonic acid dimethyl ester with n-BuLi is then reacted, thereby obtaining an alcohol (2).

The resulting alcohol is reacted with an excess of the pyridinium salt of p-toluenesulfonic acid in toluene at 80° C, thereby obtaining a cyclized product (3). This cyclized product is debenzylated by hydrogenolysis using 20% Pd(OH)₂-C under a hydrogen atmosphere (4). Using 1-ethyl-3-(3-dimethylaminopropyl)carbodiimide hydrochloride as a condensing agent, the product is reacted with a fatty acid, thereby obtaining a coupling product (5). Next, the methyl group is regioselectively removed using bromotrimethylsilane as a nucleophile, thereby obtaining a cyclic phosphonic acid (6). This is transferred to a separatory funnel using ether, and a small amount of 0.02N aqueous sodium hydroxide solution is added dropwise to separate the liquids, and the target compound is extracted and purified as the sodium salt (7).

The compound represented by Formula (2) can be produced, for example, according to the method described in JP 2021-80227 A. Specifically, a carbacyclic phosphatidic acid derivative having the following structure: 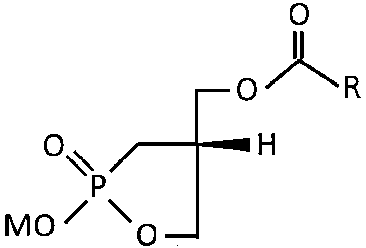 (where R and M are defined as in Formula (2))
is dissolved in an appropriate buffer (such as phosphate buffer), and the solution is diluted with artificial gastric fluid (e.g., sodium chloride solution (2 g of NaCl, 7 mL of 12 M HCl/1000 mL of water, pH 1.2)). The compound represented by Formula (2) can be produced by incubating the solution obtained above.

Alternatively, the compound of Formula (2) can be prepared using autotaxin (ATX). ATX and the carbacyclic phosphatidic acid derivative having the above structure are dissolved in an appropriate buffer solution (e.g., Tris buffer solution) to a predetermined concentration. The compound represented by Formula (1) can be produced by incubating the resulting solution at 37°C to yield a reaction.

Cyclic phosphatidic acid, carbacyclic phosphatidic acid, thiacyclic phosphatidic acid, or carba-lysophosphatidic acid, or a salt thereof, used as an active ingredient in the present invention, has the effect of suppressing the progression of dementia, primarily Alzheimer's disease, and alleviating the symptoms, and therefore can be used as an agent for ameliorating or treating dementia. In particular, cyclic phosphatidic acid, carbacyclic phosphatidic acid, thiacyclic phosphatidic acid, carba-lysophosphatidic acid, or a salt thereof used as an active ingredient in the present invention has a clear effect of ameliorating or treating dementia, primarily Alzheimer's dementia, and can therefore be used as an extremely effective agent for treating dementia.

Examples of diseases to which the present invention is applied for ameliorating or treating dementia include, but are not limited to, vascular dementia and frontotemporal dementia, in addition to Alzheimer's dementia. The agent for treating injury of the present invention is particularly useful as an agent for treating Alzheimer's dementia.

The agent for treating dementia of the present invention is preferably provided in the form of a pharmaceutical composition containing one or more pharmaceutically acceptable additives for formulation and, as an active ingredient, cyclic phosphatidic acid, carbacyclic phosphatidic acid, thiacyclic phosphatidic acid, carba-lysophosphatidic acid (preferably a compound represented by Formula (1) or (2)), or a salt thereof.

The agent for treating dementia of the present invention can be administered in various forms, and suitable administration forms may include oral administration and parenteral administration (e.g., intravenous, intramuscular, subcutaneous, or intradermal injection, rectal administration, transmucosal administration, or the like).

Examples of a pharmaceutical composition suitable for oral administration include, for example, tablets, granules, capsules, powders, solutions, suspensions, and syrups, and pharmaceutical compositions suitable for parenteral administration include, for example, injections, drip infusions, suppositories, and transdermal absorption agents; however, the dosage forms of the agent for treating dementia of the present invention are not limited to these. Furthermore, it can also be made into a sustained release preparation by known techniques. For example, a sustained-release preparation can be prepared by encapsulating cyclic phosphatidic acid, carbacyclic phosphatidic acid, thiacyclic phosphatidic acid, carba-lysophosphatidic acid, or a salt thereof as an active ingredient in a gelatin-based hydrogel.

The types of formulation additives used in producing the agent for treating dementia of the present invention are not particularly limited and can be appropriately selected by those skilled in the art. For example, an excipient, a disintegrant or disintegration aid, a binder, a lubricant, a coating agent, a base, a solubilizer or solubilizer aid, a dispersant, a suspending agent, an emulsifier, a buffer, an antioxidant, a preservative, an isotonicity agent, a pH adjuster, a solubilizer, a stabilizer, and the like can be used, and the individual specific components used for these purposes are well known to those skilled in the art.

Examples of pharmaceutical additives that can be used in preparing formulations for oral administration include: an excipient such as glucose, lactose, D-mannitol, starch, or crystalline cellulose; a disintegrant or disintegration aid such as carboxymethylcellulose, starch, or carboxymethylcellulose calcium; a binder such as hydroxypropylcellulose, hydroxypropylmethylcellulose, polyvinylpyrrolidone, or gelatin; a lubricant such as magnesium stearate or talc; a coating agent such as hydroxypropylmethylcellulose, sucrose, polyethylene glycol, or titanium oxide; and a base such as petrolatum, liquid paraffin, polyethylene glycol, gelatin, kaolin, glycerin, purified water, or hard fat.

Examples of pharmaceutical additives that can be used in preparing formulations for injection or infusion include: solubilizers or solubilizing aids that can constitute aqueous or ready-to-use injections, such as distilled water for injection, physiological saline, propylene glycol, and surfactants; isotonicity agents, such as glucose, sodium chloride, D-mannitol, and glycerin; and pH adjusters, such as inorganic acids, organic acids, inorganic bases, and organic bases.

The agent for treating dementia of the present invention can be administered to mammals such as humans.

The dosage of the agent for treating injury of the present invention should be increased or decreased as appropriate, depending on conditions such as the patient's age, sex, weight, symptoms, and route of administration. However, in general, the amount of active ingredient per day for an adult is in a range of about 1 µg/kg to 100 mg/kg, and preferably about 10 µg/kg to 10 mg/kg. The above-mentioned dose of the agent for treating dementia may be administered once or in divided doses (e.g., about 2 to 4 times) daily.

The present invention is specifically explained with reference to the Examples below; however, the present invention is not limited to the Examples.

### Examples

### Example 1:

### (1) Test Compound

The carba-cPA used in the experiment was 2ccPA (18:1). The structure of 2ccPA (18:1) is shown below. Here, -C₁₇H₃₃ represents -(CH₂)₇CH=CH(CH₂)₇CH₃ (cis form). 2ccPA (18:1) was synthesized by the method described in JP 2004-010582 A.

In addition, the carbohydrate phosphatidic acid used in the experiment was 2cLPA (18:1). The structure of 2cLPA (18:1) is shown below. Here, -C₁₇H₃₃ represents -(CH₂)₇CH=CH(CH₂)₇CH₃ (cis form). 2cLPA (18:1) was synthesized by the method described in JP 2004-010582 A.

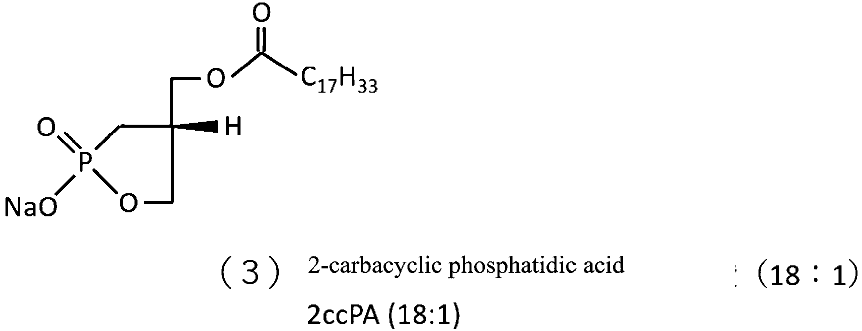

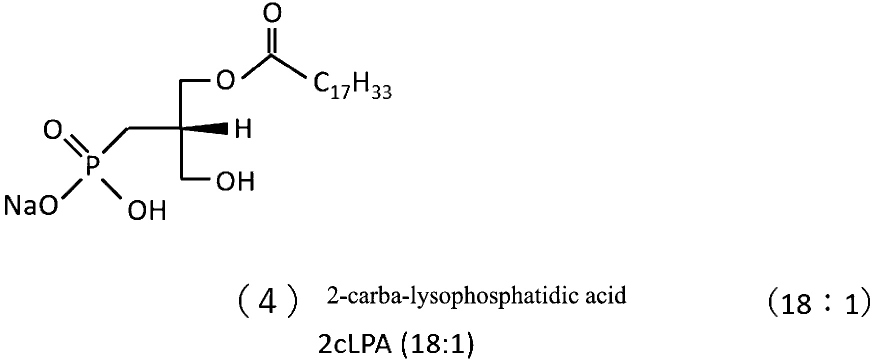

### (2) Mouse

### (2 - 1) Alzheimer's Dementia Model Mouse (pathological mouse)

Sixty-eight APPosk (SPF) male mice were produced according to the method described in Tomiyama T., Nagata T., Shimada H., Teraoka R., Fukushima A., Kanemitsu H., Takuma H., Kuwano R., Imagawa M., Ataka S., Wada Y., Yoshioka E., Nishizaki T., Watanabe Y., Mori H. A new amyloid β variant favoring oligomerization in Alzheimer's-type dementia. Ann. Neurol. 63, 377-387 (2008) at BioSafety Research Center Inc. (BSRC), which has a track record in producing model mice, and were raised until 24 weeks of age. These mice were then delivered to New Drug Research Center, Inc. and acclimatized until the preliminary breeding period. Note that quarantine was carried out from the date of arrival, considered as Day 0, up to Day 7. General observations were made daily, and body weights were measured on Day 1 (the day following the arrival), Day 3, and Day 7 after the arrival. Each individual was placed in a polysulfone cage and kept in an animal breeding room under conditions of a temperature of 22°C ± 3°C, a humidity of 50% ± 20%, and a 12-hour light/dark cycle.

The experiment was carried out in accordance with the following laws, standards, and guidelines.
- Act on Welfare and Management of Animals [Act No. 105 of October 1, 1973 (Last revised on June 19, 2019: Act No. 39 of 2019)]
- Guideline for Raising and Keeping of Laboratory Animals [Ministry of the Environment Notification No. 88, April 28, 2006 (Last revised on August 30, 2013: Ministry of the Environment Notification No. 84)]
- Guidelines on Methods of Sacrificing Animals [Notice No.40 of the Prime Minister's Office, July 4, 1995 (Partially revised on November 12, 2007: Ministry of the Environment Notification No. 105)]

This study was conducted after the Animal Experimental Protocol (Review No. 220524A) was reviewed and approved by the Institutional Animal Care and Use Committee of New Drug Research Center, Inc., the facility where the study was conducted.

### (2-2) Normal Mouse

Sixteen 24-week-old, unmodified wild-type (SPF) male mice were transported from BioSafety Research Center Inc. (BSRC), the manufacturer of the APPosk mice, to New Drug Research Center, Inc., where they were quarantined and acclimatized for seven days, with the date of arrival being considered as Day 0, as with the APPosk mice. General observations were made daily, and body weights were measured on Day 1 (the day following the arrival), Day 3, and Day 7.

The breeding environment and applicable laws, standards, and guidelines were the same as those for the APPosk mice.

### (2-3) Selection and Grouping of Animals

Only animals that showed no abnormalities during quarantine or acclimatization were used, and the animals were divided into groups according to the following group composition table, based on their body weight on Day 7 after the arrival. Of the 16 wild-type mice that arrived, 10 with weights close to the average were selected to form the normal group. Sixty-eight APPosk mice were delivered and assigned by stratified continuous randomization to the following groups: Pathological; Pathological + 2ccPA (L); Pathological + 2ccPA (H); Pathological + 2cLPA (L); and Pathological + 2cLPA (H). L: Low dose (0.9 mg/kg); H: High dose (1.8 mg/kg)

### <Group Composition Table>

**[Table 1]**

| Group | | Lineage | Dosage (mg/kg) | Number of Animals (individuals) | Animal Number |
|---|---|---|---|---|---|
| A | Normal | Wild type | 0* | 10 | 001-010 |
| B | Pathological | | 0* | 10 | 101-110 |
| C | Pathological + 2ccPA (L) | | 0.9 | 10 | 201-210 |
| D | Pathological + 2ccPA (H) | APPosk | 1.8 | 10 | 301-310 |
| E | Pathological + 2cLPA (L) | | 0.9 | 10 | 401-410 |
| F | Pathological + 2cLPA (H) | | 1.8 | 10 | 501-510 |

| | | | | | |
|---|---|---|---|---|---|
| *: Vehicle (PBS containing 0.1% BSA) was administered. | | | | | |

The animals excluded from the grouping were euthanized by laparotomy under isoflurane anesthesia and exsanguination.

### (2-5) Preparation and Administration of Dosing Solution

The substance to be administered was aliquoted and mixed with a vehicle (PBS containing 0.1% FA-free BSA).

The dosing solution was filled into a syringe (disposable syringe) equipped with a 26G needle, and administered intraperitoneally once daily for 168 days (24 weeks). On the day when the Morris water maze test was performed, the dosing solution was administered after the Morris water maze test.

### (3) Observation of general conditions and body weight measurement

### (3-1) Observation of general conditions

General conditions were observed daily and recorded. Throughout this study, no animals were found to meet humane endpoints, and no animals showed any significant abnormalities. However, temporary swelling of the urogenital area was observed in Animal Nos. 403 and 405, but both recovered within a few days.

On Day 97 after administration, the death of one animal, Animal No. 101 (pathological group), was confirmed. Since the animal had been in good health until the day before and no notable abnormalities were found during a gross autopsy examination, the cause of death was determined to be sudden and unknown.

### (3-2) Body weight measurement

Body weight was measured using an electronic balance once a week. Each time, the measurement was carried out twice at different times. No significant change in body weight was observed in 59 mice, excluding one mouse in the pathological group that died. No weight loss was observed after 24 weeks of daily administration of 2ccPA and 2cLPA, confirming the safety of both test substances. During the test period, two animals from the normal group that showed significant outliers in the Morris water maze test were excluded from the result assessment.

Table 2 shows the results of body weight measurement carried out once a week as an index of the health status of the test animals. The results demonstrate the safety of both test substances.

**[Table 2]**

| Group | | Lineage | Animal Number | Weight change (average) Standard error |
|---|---|---|---|---|
| A | Normal | Wild type | 001-010 | 30.5.-34.5 (33.0) |
| | | | | 0.5 |
| B | Pathological | | 101-110 | 30.1-33.8 (31.5) |
| | | | | 0.9 |
| C | Pathological + 2ccPA (L) | | 201-210 | 31.4-33.6 (32.3) |
| | | | | 0.7 |
| D | Pathological + 2ccPA (H) | APPosk | 301-310 | 30.6-32.8 (32.1) |
| | | | | 0.6 |
| E | Pathological + 2cLPA (L) | | 401-410 | 30.8-33.3 (32.0) |
| | | | | 0.6 |
| F | Pathological + 2cLPA (H) | | 501-510 | 29.6-31.9((30.2) |
| | | | | 1.0 |

### (4) Morris Water Maze Test

When the mice reached 12 months of age (48 weeks of age), the Morris water maze test was performed.

The water maze (diameter: 90 cm; height: 60 cm; water temperature: approximately 22°C) was made opaque with a white non-toxic paint (skim milk) and divided into four virtual quadrants (east, west, north, and south). A circular platform having a diameter of 8 cm was submerged approximately 1 cm below the water surface and placed near the center of the north quadrant. Black markers were placed on the walls of the water maze as extramaze cues.

The time it took to reach the platform placed in the water maze (escape latency) was measured over 4 days. Each animal underwent five consecutive trials, with an interval of approximately 20 seconds between the end and start of each trial.

Each trial began with the mouse being placed in any quadrant except the north quadrant, with its face facing the outer edge of the water maze. A webcam (Logicool HD Webcam C615) and SMART v3.0 (Panlab Harvard Apparatus) were used for measurement and analysis. When the mouse reached the platform within 60 seconds from the start of the measurement and remained stationary on the platform for 10 seconds, the measurement was terminated, and the mouse was returned to its breeding cage. In a case in which the mouse did not reach the platform within 60 seconds, or in a case in which the mouse reached the platform but did not remain stationary on the platform for 10 seconds, the measurement was terminated within 70 seconds from the start of the measurement. The person conducting the measurement guided the mouse to the platform, kept it on the platform for 10 seconds, and then returned it to its breeding cage.

In addition, due to the analysis settings in SMART v3.0, in a case in which the mouse did not reach the platform within 60 seconds, or in a case in which the mouse reached the platform within 60 seconds but did not remain stationary on the platform for 10 seconds, the value output would be 60 seconds or more, but all values were treated as 60 seconds. In the normal group (Group A), two individuals having an average escape latency exceeding 40 seconds in the five attempts on Day 4 of the water maze were set as wild-type, but were judged to have originally low cognitive function and were excluded from the data to calculate the average value and standard error. In addition, in the pathological group (Group B), individuals having an average escape latency of 20 seconds or less in the five attempts on Day 4 of the water maze were judged to have insufficient cognitive decline, and were planned to be excluded from the data to calculate the average value and standard error, but no such individuals were found.

### (5) Memory improvement effect in the Morris water maze test

In this study, as described above, 2ccPA/2cLPA was intraperitoneally administered to 24-week-old APPosk mice for 168 days (24 weeks), and the effects of 2ccPA/2cLPA on memory assessment were examined using the Morris water maze test.

The results of the Morris water maze test are shown below.

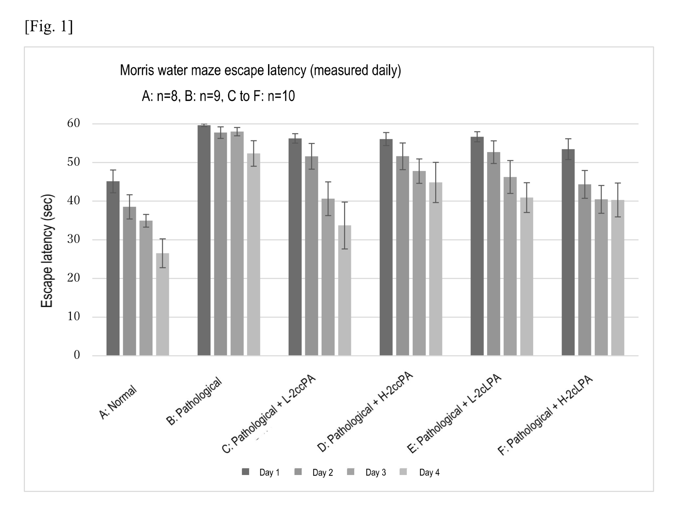

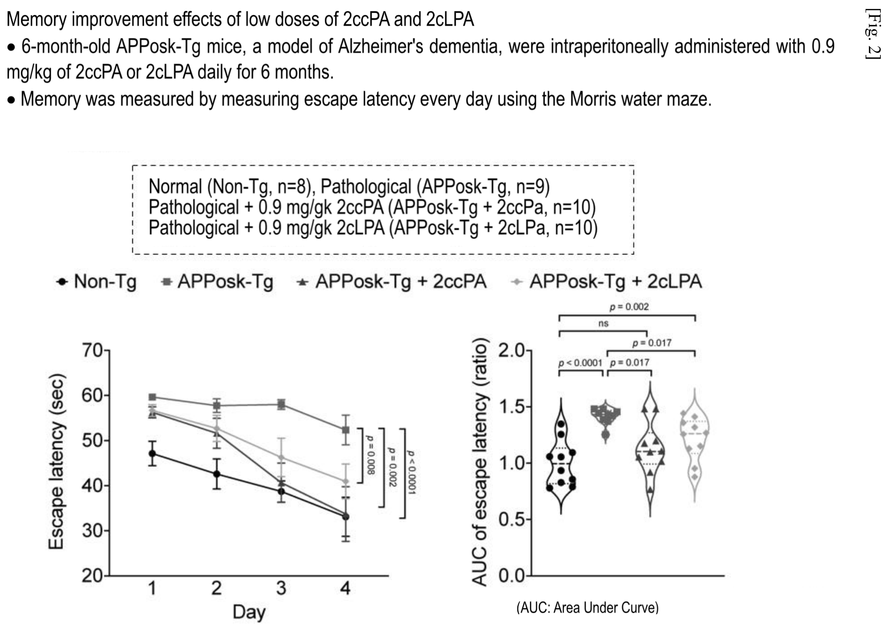

Fig. 1 shows the results of escape latency in the Morris water maze (measured five times per day) for normal, pathological, pathological + 2ccPA (L: low dose and H: high dose), and pathological + 2cLPA (L and H) mice. Fig. 2 shows the memory improvement effect at low doses of 2ccPA and 2cLPA.

As described in (4), each measurement value represents the combined results of five consecutive trials for each animal over four days. It was confirmed that administration of 2ccPA/2cLPA significantly improved memory in the model mice. In particular, a low dose of 2ccPA (0.9 mg/kg) was observed to have a memory maintenance effect comparable to that of the normal group.

These results demonstrate that both 2ccPA and 2cLPA significantly improve memory decline in Alzheimer's dementia model mice.

### (6) Autopsy

An autopsy was performed on the day after the final administration. The abdomen was opened under isoflurane anesthesia, and exsanguination was performed until death resulting from exsanguination, following whole blood collection from the posterior vena cava.

After exsanguination, 6 of the 10 individuals in each group (with numbers ending in 1 to 6) were perfused with phosphate-buffered saline (PBS) and then perfused again with 4% paraformaldehyde (PFA) to fix the brains, after which the brains were removed. The extracted brains were then infiltrated and fixed using the same fixative solution. The remaining four individuals in each group (with numbers ending in 7 to 10) were perfused whole body with PBS, and then their brains were extracted and frozen using liquid nitrogen.

For subsequent analysis, the collected blood was centrifuged to separate the serum and stored at -70°C or below, the brains fixed in 4% PFA were stored at room temperature, and the frozen brains were stored at -70°C or below.

### (7) Quantitative determination of tau protein and phosphorylated tau protein

Serial sections (thickness: 40 µm) of fixed mouse cerebral cortex were prepared using a cryostat. The sections were incubated overnight with primary antibodies and then for 1 hour with secondary antibodies. Stained images were then captured using a confocal microscope (Carl Zeiss) and quantified using NIH ImageJ. The primary antibodies used were non-phosphorylated tau antibody (Invitrogen), phosphorylated tau (Ser396) antibody (Invitrogen), and phosphorylated tau (Ser404) antibody (Invitrogen), and the secondary antibodies used were biotinylated goat anti-mouse IgG antibody (Vector Lab.) and biotinylated goat anti-rabbit IgG antibody (Vector Lab.). Quantitative determination was carried out using five sections each.

**[Table 3]**

| Quantitative determination of tau protein and phosphorylated tau protein in the hippocampus of normal, pathological, pathological + 2ccPA (low dose), and pathological + 2cLPA (low dose) mice | | | |
|---|---|---|---|
| Group | Tau protein area / Hippocampus area (%) (average) | Phosphorylated tau (Ser396) area / Hippocampus area (%) (average) | Phosphorylated tau (Ser404) area / Hippocampus area (%) (average) |
| Normal (Non-Tg) | 16.0-36.2 (**24.2**) | 0.5-4.6 (**2.2**) | 1.4-4.7 (**3.1**) |
| Pathological (APPosk-Tg) | 17.1-39.3 | 4.7-13.2 (**9.2**) | 2.1-6.3 (**3.9**) |
| | (**24.9**) | | |
| Pathological + 2ccPA (L) (APPosk-Tg+2ccPA) | 7.7-19.5 (**17.4**) | 2.0-4.9 (**2.3**) | 0.4-1.7 (**0.5**) |
| Pathological + 2cLPA (L) (APPosk-Tg+2cLPA) | 10.6-36.2 (**19.0**) | 0.5-4.7 (**2.4**) | 0.9-1.8 (**1.2**) |

These results demonstrate that both 2ccPA and 2cLPA significantly suppress the phosphorylation of tau protein, which is thought to be involved in the development of dementia.

### (8) Evidence that 2ccPA crosses the blood-brain barrier: Application of microdialysis

To confirm that 2ccPA crosses the blood-brain barrier (BBB) and enters the brain, an experiment was conducted at the Kurume Modality Analysis Laboratory of BioSafety Research Center Inc. (BSRC), which has a long track record in microdialysis, and the Institute for Disease Modeling, Kurume University School of Medicine.

The regulations concerning animal experiments, which were followed herein, are as follows:
- "Act on Welfare and Management of Animals" [Act No. 105 of October 1, 1973; Last revised on June 19, 2019: Act No. 39]
- "The Guideline for Raising and Keeping of Laboratory Animals" (Ministry of the Environment Notification No. 88 of April 28, 2006; Last revised on August 30, 2013: Ministry of the Environment Notification No. 84)

This study was also reviewed and approved by the Institutional Animal Care and Use Committee of Kurume University before its commencement (Approval No. 2023-189).

2ccPA was administered intraperitoneally to 33-week-old male C57BL/6J-Aged mice (SPF, Jackson Laboratory Japan, Inc.) with a dialysis probe (FX-I-4-02, EICOM) placed in the hippocampus of the mouse brain. After that, the brain microdialysis perfusate was collected at 10 time points, 1 hour before administration and every 30 minutes (0.5 to 5.0 hours) after administration, using the *in vivo* microdialysis method.

The hippocampal guide cannula placement surgery was performed under isoflurane anesthesia, with a guide cannula and a dummy cannula placed at the hippocampal coordinate position (AP = -3.1 mm; ML = +2.7 mm; DV = -3.7 mm). After surgery, Vetorphale was administered subcutaneously for analgesia. Three to five days after the cannula was placed, the cannula was replaced with a dialysis probe under isoflurane inhalation anesthesia, and pre-perfusion (perfusion solution: saline containing 0.15% BSA) was then initiated.

2ccPA administration was performed under isoflurane anesthesia. D-PBS(-) containing 0.1% BSA was added to 2 cc of weighed PA, and the mixture was dispersed by ultrasonic treatment, thereby preparing a dosing solution.

The dosing solution was diluted with D-PBS(-) containing 0.1% BSA to a concentration of 0.9 mg/kg/day, and then sonicated individually just immediately administration. The administration route was intraperitoneal administration.

The microdialysis perfusate was collected at 11 time points: 1 hour before administration and 0.5, 1.0, 1.5, 2.0, 2.5, 3.0, 3.5, 4.0, 4.5, and 5.0 hours after administration, and each was collected into ice-cooled siliconized tubes.

Five mice in each test group underwent hippocampal cannula placement surgery, replacement with a dialysis probe, administration of the test substance, and collection of the microdialysis perfusate. The collected solution was added to an ice-cold acid-methanol solution (pH 4), stirred, and then promptly frozen in liquid nitrogen and stored at -80°C.

### (9) Evidence that 2ccPA crosses the blood-brain barrier (BBB): Quantitative determination of microdialysis recovery fluid by LC/MS

The concentration of 2ccPA in the perfusate collected by microdialysis from mice administered with 2ccPA was measured using LC/MS. The conditions are as follows: Shimizu Y., Fukasawa K., Yamamoto S., Shibike Y., Tsukahara R., Ishikawa M., Iwasa K., Yoshikawa K., Gotoh M., Murakami-Murofushi K. Evaluation of the pharmacokinetics of 2-carba-cyclic phosphatidic acid by liquid chromatography-triple quadrupole mass spectrometry

### Prostaglandins Other Lipid Mediat.150:106450. doi:10.1016(2020).

The standard solution for the calibration curve was prepared by adding ethanol to the standard substance, 2ccPA, dissolving it, and then mixing it with the blank matrix. The internal standard solution (IStd) was prepared by adding ultrapure water to the internal standard substance (ranitidine hydrochloride), dissolving it, and then diluting it with ultrapure water (10 ng/mL). The measurement specimen (microdialysis perfusate) was prepared by adding an acidic methanol solution (pH 4) in an amount five times the volume of the sample.

The measurement samples (the measurement specimen and the standard solution for the calibration curve) were deproteinized by adding a specimen treatment solution containing the internal standard solution to each sample. After centrifugation, the supernatant was filtered, and the 2ccPA concentration was measured using a Q Exactive Ultimate 3000 system (Thermo Scientific), thereby yielding the following results.

**[Table 4]**

| 2cc PA concentration in microdialysis recovery fluid | | | | | | | | | | | |
|---|---|---|---|---|---|---|---|---|---|---|---|
| After administration (Time) | 0 | 0.5 | 1.0 | 1.5 | 2.0 | 2.5 | 3.0 | 3.5 | 4.0 | 4.5 | 5.0 |
| 2ccPA concentration (ng/mL) | 0 | 1.90 | 2.22 | 2.81 | 2.24 | 1.64 | 1.80 | 2.32 | 2.12 | 2.12 | 1.22 |

These results indicated that the administered 2ccPA passed through the barrier of endothelial cells that line the walls of brain capillaries and acted on brain tissue. 2ccPA acts directly on the brain and is expected to be effective in treating a variety of neurological diseases, including Alzheimer's disease (AD).

## Claims

1. An agent for ameliorating or treating dementia, which comprises cyclic phosphatidic acid, carbacyclic phosphatidic acid, thiacyclic phosphatidic acid, or carba-lysophosphatidic acid, or a salt thereof.

2. The agent for ameliorating or treating dementia according to claim 1, wherein the dementia is Alzheimer's dementia.

3. The agent for ameliorating or treating dementia according to claim 1 or 2, wherein the cyclic phosphatidic acid, carbacyclic phosphatidic acid, or thiacyclic phosphatidic acid, or a salt thereof is a compound represented by Formula (1), and the carba-lysophosphatidic acid or a salt thereof is a compound represented by Formula (2): 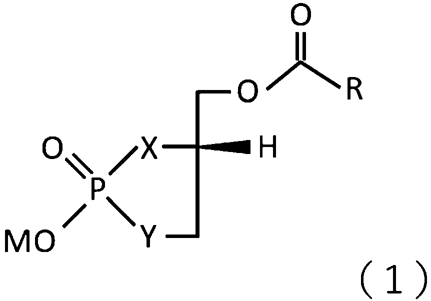 (in Formula (1), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring; X and Y each independently represent -O-, -S- or -CH₂-, provided that X and Y are not simultaneously -CH₂-; and M is a hydrogen atom or a counter cation) 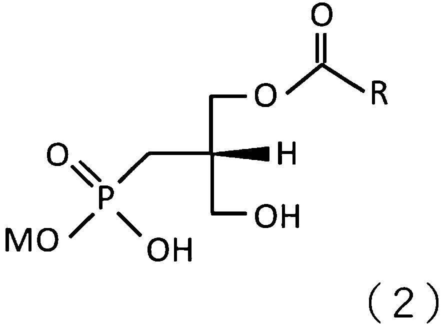 (in Formula (2), R represents a linear or branched alkyl group having 1 to 30 carbon atoms, a linear or branched alkenyl group having 2 to 30 carbon atoms, or a linear or branched alkynyl group having 2 to 30 carbon atoms, and these groups may include a cycloalkane ring or an aromatic ring; and M is a hydrogen atom or a counter cation).

4. The agent for ameliorating or treating dementia according to claim 3, wherein one of X and Y is -CH₂-, and the other of X and Y is -O- in Formula (1).
